# EUROPEAN PATENT SPECIFICATION

(11) **EP 0 484 063 B1**
(45) Date of publication and mention of the grant of the patent: **06.03.1996**
(21) Application number: 91309880.2
(22) Date of filing: 25.10.1991
(51) Int. Cl.: C12P 41/00, C12P 17/02, C07D 301/00

(54) **Process for preparing optically active epoxy alcohols**
Verfahren zur Herstellung von optisch aktiven Epoxyalcoholen
Procédé pour la préparation d'époxy-alcools optiquement actifs

(30) Priority: 30.10.1990 JP 294486/90; 14.06.1991 JP 170625/91
(43) Date of publication of application: 06.05.1992
(73) Proprietor: Nitto Denko Co. Ltd., Ibaraki City, Osaka 567 (JP)
(72) Inventor: Fukusaki, Eiichiro, Nitto Denko Co. Ltd., Ibaraki City, Osaka 567 (JP); Takahama, Hiroshi, Nitto Denko Co. Ltd., Ibaraki City, Osaka 567 (JP); Hada, Minako, Nitto Denko Co. Ltd., Ibaraki City, Osaka 567 (JP); Kimura, Yasuyuki, Nitto Denko Co. Ltd., Ibaraki City, Osaka 567 (JP); Yuasa, Hiroyuki, Nitto Denko Co. Ltd., Ibaraki City, Osaka 567 (JP); Nakazono, Yutaka, Nitto Denko Co. Ltd., Ibaraki City, Osaka 567 (JP); Senda, Shuji, Nitto Denko Co. Ltd., Ibaraki City, Osaka 567 (JP); Omata, Tetsuo, Nitto Denko Co. Ltd., Ibaraki City, Osaka 567 (JP)
(74) Representative: Copp, David Christopher

(56) References cited:
- EP-A- 0 344 791
- EP-A- 0 345 658
- EP-A- 0 357 009
- JP-A-01 171 497
- JP-A-01 235 599
- US-A- 4 732 853
- US-A- 4 923 810
- JOURNAL OF THE AMERICAN CHEMICAL SOCIETY. vol. 106, 1984, GASTON, PA US pages 7250 - 7251; W.E.LADNER ET AL.: 'Lipase-catalyzed hydrolysis as a route to esters of chiral epoxy alcohols'
- TETRAHEDRON LETTERS. vol. 29, no. 20, 1988, OXFORD GB pages 2455 - 2458; DANIELE BIANCHI ET AL.: 'Enzymatic resolution of 2,3-epoxyalcohols, intermediates in the synthesis in the Gypsy moth sex pheromone.'
- CHEMICAL ABSTRACTS, vol. 112, no. 19, 7 May 1990, Columbus, Ohio, US; abstract no. 177061, AMANO, MASAKI ET AL.: 'Optical resolution of racemic alcohols with hydrolase' page 595 ;
- "A Guidebook to Mechanism in Organic Chemistry", Sykes, 4th edition 1974, pp 42 - 43, 2.2.3

## Description

The present invention relates to a process for preparing optically active epoxy alcohols.

The optically active epoxy alcohol compounds of formula 1 are noted as synthetic intermediates of medicines, pesticides, and biologically active substances.

Especially the (-)optically active compound 2,3-epoxy-8-methyl-1-nonanol is an important constituent of the sex pheromone of Lymantria dispar L. 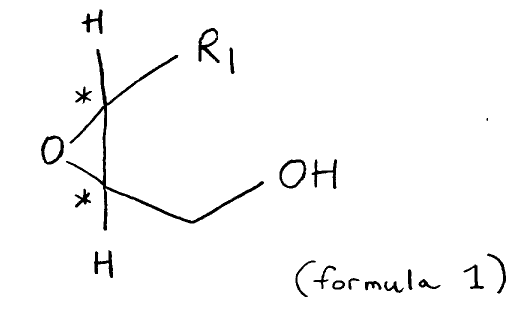 (Here R₁ denotes a straight-chain or branched alkyl group containing 1 through 20 carbon atoms.)

Synthesis of an epoxy alcohol of formula 1 has been proposed by Katsuki K. (J.A. Chem. Soc. 1980, vol 102; 5,974) which utilizes asymmetric epoxidation of an allyl alcohol.

This method is, however not practical since it suffers from the problems that:
(1) It requires an expensive asymmetric reducing agent;
(2) the temperature has to be controlled to below -50°C; and
(3) it requires the use of dangerous agents such as t-butyl hydroperoxide.

Another known method proposed by Daniel Bianchi (tetraheron Letter, 29 (20) 2455 to 2458, 88) is to synthesize the optically active epoxy alcohol compound of formula 1 by means of asymmetric transesterification of an epoxy alcohol of formula 1 and acetates thereof with lipase. This method also has problems in practical application such as low optical purity and poor yield.

Other workers have prepared the (+) form of an epoxy alcohol of formula 1 by selective hydrolysis of a racemic ester mixture in an aqueous or aqueous/organic solvent in the presence of a hydrolase. See, for example, US patent numbers 4 732 853, 4 923 810, and J Am Chem Soc 1984, 106, 7250-7251.

It is known from Japanese patent application 1-235599 to irreversibly and preferentially esterify one antipode of a racemic alcohol with a carboxylic anhydride in an organic solvent in the presence of a hydrolase. The method has been used successfully with some secondary alcohols. Japanese patent application 1-171497 relates to a similar method, but esterifying with an enol ester of a carboxylic acid.

The object of the present invention is to provide a process for the preparation of an epoxy alcohol which readily, selectively and efficiently yields the (-) form of optically active epoxy alcohols of formula 1 which are useful synthetic intermediates of medicines, pesticides and biologically active substances such as the sex pheromone of Lymantria dispar L.

According to the present invention there is provided a process for preparing optically active epoxy alcohol, which is characterised in that it comprises the steps of:
adding carboxylic anhydride to racemic epoxy alcohol of formula 1 in the presence of a hydrolase in an organic solvent;
esterifying (-)-form of the epoxy alcohol preferentially to give an epoxy ester of formula 2;
separating optically active epoxy ester of formula 2 from optically active epoxy alcohol of formula 3; and
hydrolyzing said optically active epoxy ester of formula 2 to yield optically active epoxy alcohol of formula 4;
whereby said optically active epoxy alcohol of formula 4 is synthesised in two steps, a first step being a hydrolase enzyme reaction to prepare an optically active ester from a racemic alcohol, and a second step being a hydrolysis reaction to prepare optically active alcohol from said optically active ester. 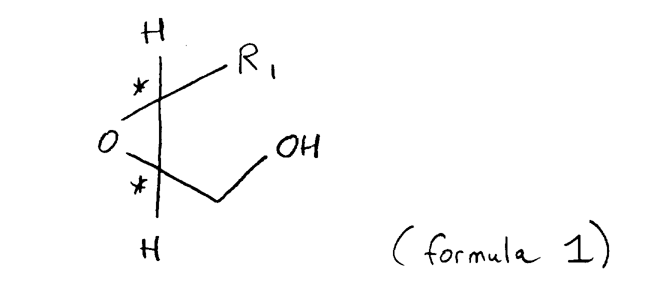 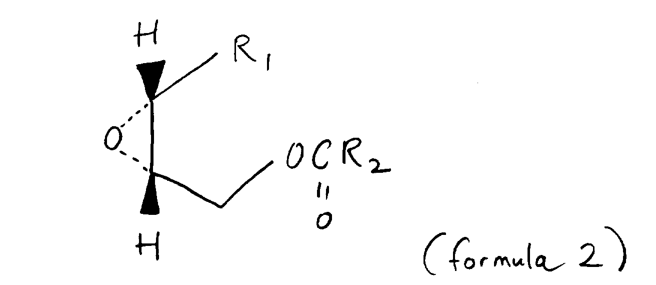 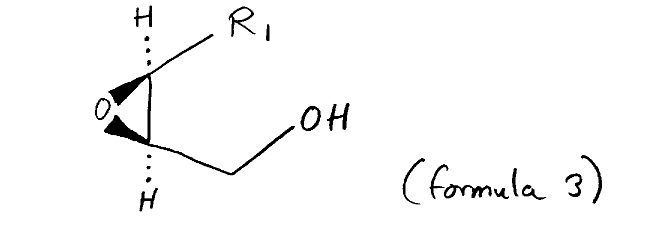 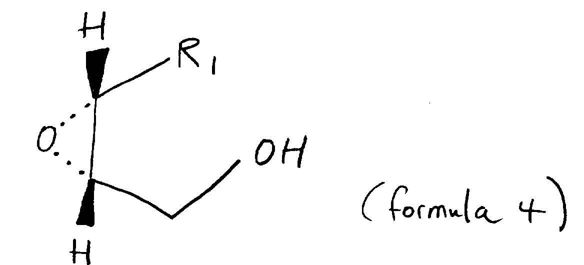 (Here, R₁ and R₂ denote straight-chain or branched alkyl groups containing 1 through 20 carbon atoms, * denotes asymmetric carbon atoms.)

The invention will now be described in more detail.

The racemic epoxy alcohol of formula 1, the starting material in this invention, is easily obtained or prepared.

For example, allyl alcohol of formula 5 can be reacted with the vanadyl acetylacetonate and t-butyl hydroperoxide. 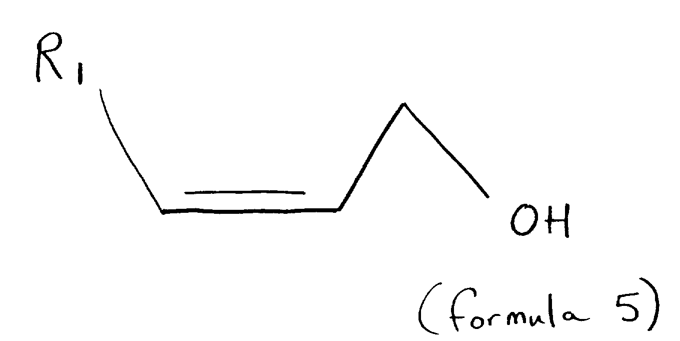 (Here, R₁ denotes a straight-chain or branched alkyl group containing 1 through 20 carbon atoms.)

Among the embodiments of the epoxy alcohol, especially useful is the (-)optically active compound 2, 3-epoxy-8-methyl-1-nonanol, useful as the synthetic intermediate of sex pheromone for injurious insects in forests (Lymantria dispar L.)

That is the Lymantria dispar L. as prepared from (-)-(2S,3R)-2,3-epoxy-8-methyl-1-nonanol by the method of K Mori and others (Tetrahedron; vol 42 3471 (1986)).

In the process of this invention, the carboxylic anhydride is added to the epoxy alcohol of formula 1 in the presence of a hydrolase in an organic solvent so as to acylate (-)optically active substance of epoxy alcohol preferentially so as to give optically active epoxy ester of formula 2. 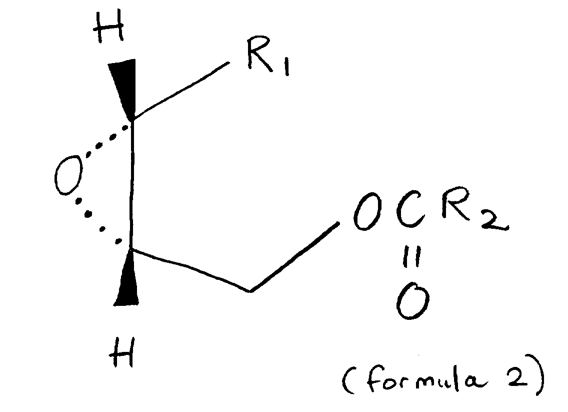 (Here R₁ and R₂ denote straight-chain or branched alkyl groups containing 1 through 20 carbon atoms.)

In this reaction the enzyme to be used can be a lipase such as a lipase derived from pancreas of pig, yeast, mold or an esterase such as an esterase derived from liver of pig, a cholesterolesterase.

Either purified or crude enzymes can be used, and there is no restriction on the state of the enzymes, therefore any state such as powder, grain or dried biomass microorganism (treateted biomass and paused biomass) containing the enzyme can be used.

These enzymes can be used as they are, or immobilised on a carrier.

Moreover after this reaction, it is possible to re-use the recovered enzymes.

The organic solvent favourably used in this reaction can be any non-aqueous solvents, for example an acyclic hydrocarbon solvent such as n-hexane, n-heptane, n-octane, isobutane,isopentane, isooctane or a cyclic hydrocarbon solvent such as cyclopentane, cyclohexane or a halogenated hydrocarbon solvent such as dichloromethane, trichloromethane or an aromatic hydrocarbon solvent such as benzene, toluene, xylene or an ether group solvent such as diethylether, diisopropylether, n-butylether, tetrahydrofuran, tetrahydropyran and carbon tetrachloride.

All of the carboxylic acids which can be a substrate for hydrolase can be used, and preferred acids are acyclic carboxylic acids with 2 to 10 carbon atoms or cyclic carboxylic acids with 4 to 10 carbon atoms.

Examples of acyclic carboxylic acids are acetate, acetic acid, propionic acid, butyric acid, valeric acid or capronic acid, and cyclic carboxylic acid anhydrides such as succinic anhydride, maleic anhydride and glutaric anhydride.

The ratio of combination of racemic epoxy alcohol of formula 1: acyl group donator (carboxylic anhydride) is 1: not less than 0.5 in mole ratio.

The reaction temperature is preferably within the active temperature for the enzyme and ordinarily it is the range 10°C to 50°C.

The obtained optically active (-)-epoxy esters of formula 2 are oily materials at room temperature.

After the asymmetric hydrolysis reaction, optically active (-)-epoxy ester (formula 2) and optically active(+)-epoxy alcohol (formula 3) are separated from the reaction mixture. 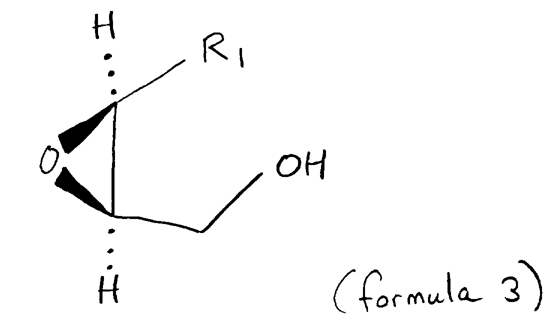 (Here R₁ denotes a straight-chain or branched alkyl group containing 1 through 20 carbon atoms.)

Examples of this separating process are extraction by using organic solvent being slightly soluble in water, two solvents being an insoluble organic solvent and water as a two layer system and distillation.

Moreover the optically active(-)-epoxy ester of formula 2 obtained from this separation can be easily converted into optically active (-)-epoxy alcohol of formula 4 by hydrolyzing with alkali such as potassium hydroxide. 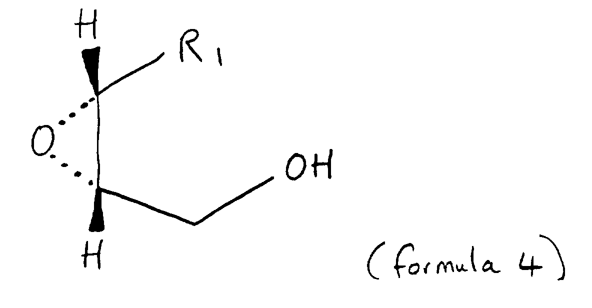 (Here R₁ denotes a straight-chain or branched alkyl group containing 1 through 20 carbon atoms.)

As described above, in this invention, optically active (+)-epoxy alcohol (formula 3) and optically active (-)-epoxy alcohol (formula 4) are obtained.

The process of preparing optically active epoxy alcohols from the intermediate optically active epoxy ester of formula 2 is novel.

The process for preparing optically active epoxy alcohol according to this invention as described above is effective to obtain the optically active substances in a high optical purity of more than 80% e.e easily and extremely safely at ordinary temperature.

The infra red and proton nuclear magnetic resonance spectras of certain epoxy alcohols and their esters are shown in the accompanying drawings, in which:
Figure 1 is an infra red absorption spectrum of (-)-(2S,3R)-1-acyloxy-2,3-epoxy-8-methylnonane.
Figure 2 is a proton nuclear magnetic resonance spectrum of (-)-(2S,3R)-1-acyloxy-2,3-epoxy-8-methylnonane.
Figure 3 is an infra red absorption spectrum of (-)-(2S,3R)-1-propionyloxy-2,3-epoxy-8-methylnonane.
Figure 4 is a proton nuclear magnetic resonance spectrum of (-)-(2S,3R)-1-propionyloxy-2,3-epoxy-8-methylnonane.
Figure 5 is a infra red absorption spectrum of (-)-(2S,3R)-1-butyroxy-2,3-epoxy-8-methylnonane.
Figure 6 is a proton nuclear magnetic resonance spectrum of (-)-(2S,3R)-1-butryoxy-2,3-epoxy-8-methylnonane.
Figure 7 is a infra red absorption spectrum of (-)-(2S,3R)-1-valeroxy-2,3-epoxy-8-methylnonane.
Figure 8 is a proton nuclear magnetic resonance spectrum of (-)-(2S,3R)-1-valeroxy-2,3-epoxy-8-methylnonane.
Figure 9 is a infra red absorption spectrum of (-)-(2S,3R)-1-capryloxy-2,3-epoxy-8-methylnonane.
Figure 10 is a proton nuclear magnetic resonance spectrum of (-)-(2S,3R)-1-capryloxy-2,3-epoxy-8-methylnonane.

This invention will now be described by means of the following Examples.

### Example 1

Toluene (50ml), 2,3-epoxy-8-methyl-l-nonanol (5g) and acetic anhydride (2g) were mixed in a 100ml Mayer flask.

Lipase originated from pig pancreas (5g) [Trade name: Pancreatin F®, Maker: Amano Pharmaceutical] was added to the mixed solution and stirred with a magnetic stirrer at 25°C for four hours for conclusion of the reaction.

After the solution was filtered, the filtrate was concentrated with a rotary evaporator and the solvent was removed.

The oily substance thus obtained was separated by silica gel column chromatography with hexane/ethyl acetate as eluate to yield 3.2g of (-)-(2S,3R)-1-acyloxy-2,3-epoxy-8-methylnonane (optical purity : 75% e.e) and 2.3g of (+)-(2R,3S)-2,3-epoxy-8-methyl-1-nonanol (optical purity : 85% e.e).

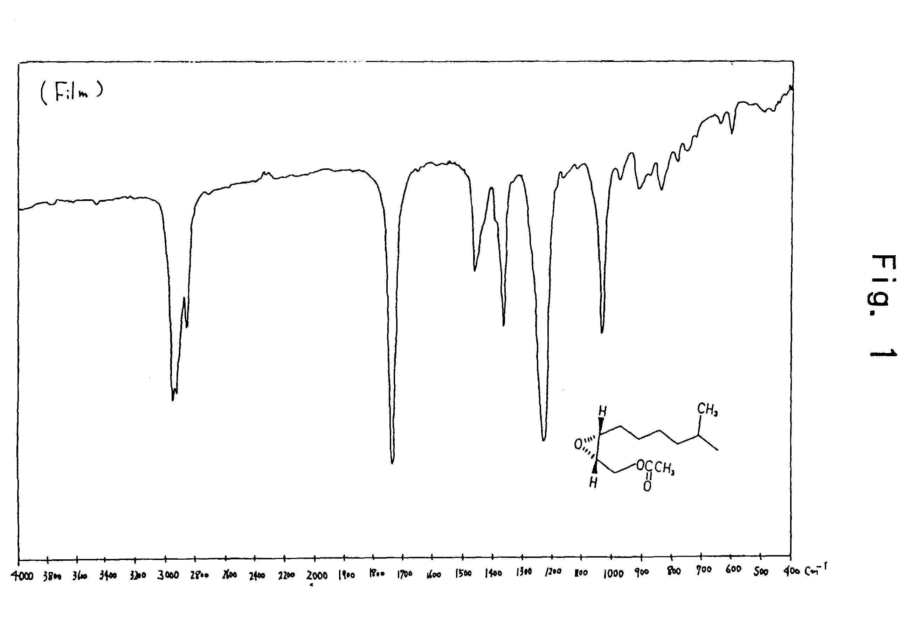

The infra red absorption spectra of (-)-(2S,3R)-1-acyloxy-2,3-epoxy-8-methylnonane showed the absorption at wavelengths (cm⁻¹ of 2,950(s), 2,930(s), 2,860(s), 1,740(s), 1,460(m), 1,360(m), 1,230(s), and 1,030(s) (see Fig. 1).

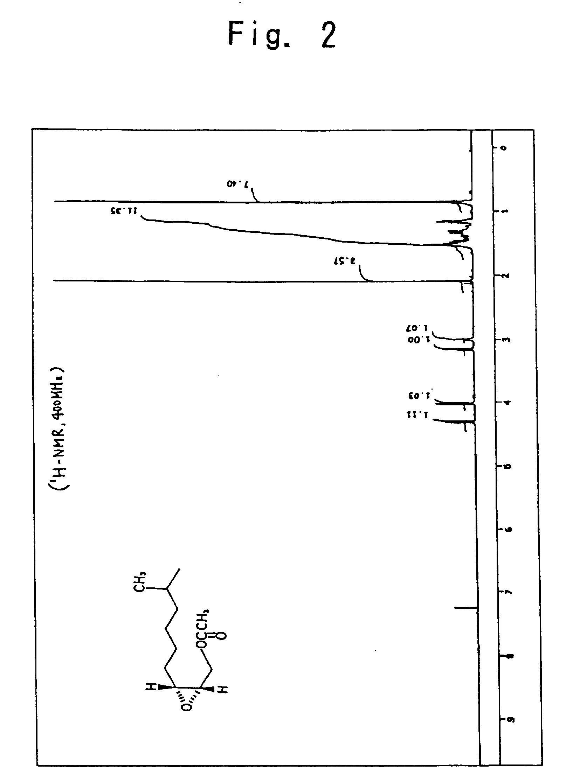

The δ values of the proton nuclear magnetic resonance spectra ¹H-NMR (CDC1₃, 400 MHz) were 0.85 (6H, d, J = 6.9Hz), 1.13 through 1.54 (9H, m), 2.08 (3H, s), 2.99 (1H, m), 3.15 (1H, m), 4.01 (1H, m) and 4.30 (1H, m) (see Fig. 2).

3.2g of (-)-(2S,3R)-1-acyloxy-2,3-epoxy-8-methylnonane was hydrolysed with potassium hydroxide/methanol to yield 2.4g of (-)-(2S,3R)-2,3-epoxy-8-methyl-1-nonanol.

Benzoyl chloride was added to the compound to give benzoyl ester. Then the optical purity thereof was measured with an HPLC column for optical separation [Trade name : chiralcel OJ by Dicell Chemical Industries] (other examples were also measured in the same manner).

### Example 2

Toluene (50ml), 2,3-epoxy-8-methyl-1-nonanol (5g) and propionic anhydride (2g) were mixed in a 100ml Mayer flask.

Lipase originated from pig pancreas (5g) [Trade name : Pancreatin F, Maker : Amano Pharmaceutical] was added to the mixed solution and stirred with a magnetic stirrer at 25°C for four hours for conclusion of the reaction.

After the solution was filtered, the filtrate was concentrated with a rotary evaporator and the solvent was removed.

The oily substance thus obtained was separated by silica gel column chromatography with hexane/ethyl acetate as eluate to yield 3.4g of (-)-(2S,3R)-1-propionyloxy-2,3-epoxy-8-methylnonane (optical purity : 80% e.e) and 2.2g of (+)-(2R,3S)-2,3-epoxy-8-methyl-1-nonanol (optical purity: 88% e.e).

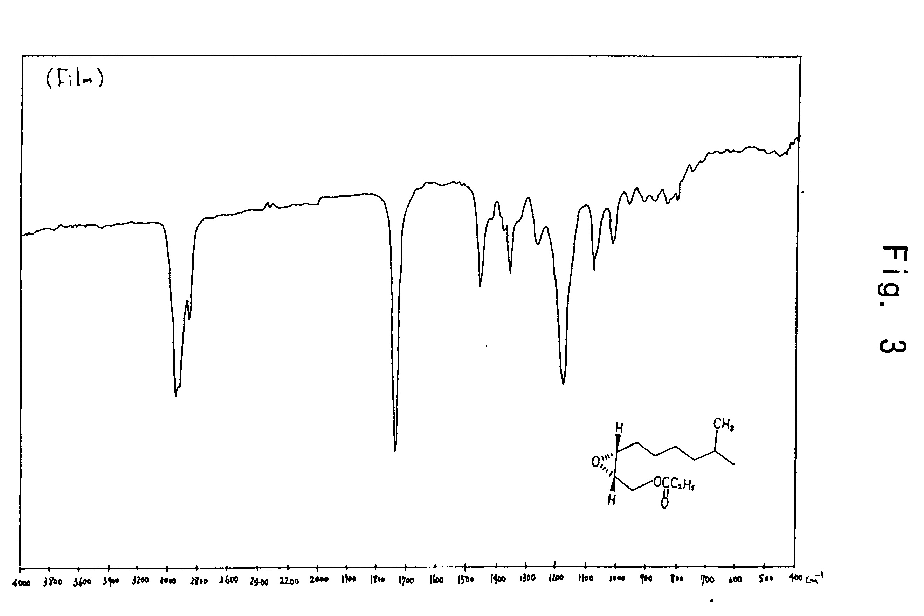

The infra red absorption spectra of (-)-(2S,3R)-1-propionyloxy-2,3-epoxy-8-methylnonane showed the absorption at wavelengths (cm⁻¹ of 2,950(s), 2,920(s), 2,850(s), 1,740(s), 1,460(m), 1,360(m), 1,180(s), and 1,080(s) (see Fig. 3).

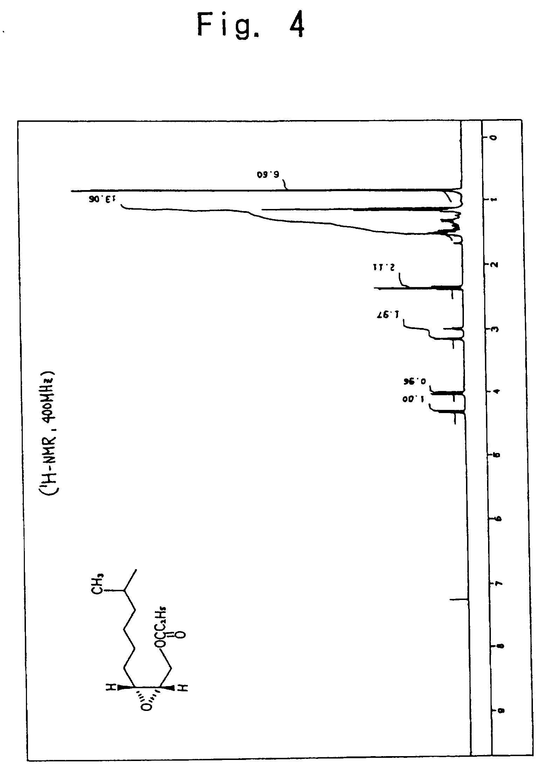

The δ values of the proton nuclear magnetic resonance spectra ¹H-NMR (CDC1₃, 400 MHz) were 0.84 (6H, d, J = 6.9Hz), 1.12 through 1.54 (12H, m), 2.36 (2H, q, J = 7.6Hz), 2.98 (1H, m), 3.14 (1H, m), 4.02 (1H, m) and 4.30 (1H, m) (see Fig. 4).

3.4g of (-)-(2S,3R)-1-propionyloxy-2,3-epoxy-8-methylnonane was hydrolized with potassium hydroxide/methanol to yield 2.4g of (-)-(2S,3R)-2,3-epoxy-8-methyl-1-nonanol.

### Example 3

Toluene (50ml), 2,3-epoxy-8-methyl-1-nonanol (5g), and n-butyric acid anhydride (3g) were mixed in a 100ml Mayer flask.

Lipase originated from pig pancreas (5g) [Trade name : Pancreatin F, Maker : Amano Pharmaceutical] was added to the mixed solution and stirred with a magnetic stirrer at 25°C for three hours for conclusion of the reaction.

After the solution was filtered, the filtrate was concentrated with a rotary evaporator and the solvent was removed.

The oily substance thus obtained was separated by silica gel column chromatography with hexane/ethyl acetate as eluate to yield 3.6g of (-)-(2S,3R)-1-butyroxy-2,3-epoxy-8-methylnonane (optical purity : 89% e.e) and 2.2g of (+)-(2R,3S)-2,3-epoxy-8-methyl-1-nonanol (optical purity: 91% e.e).

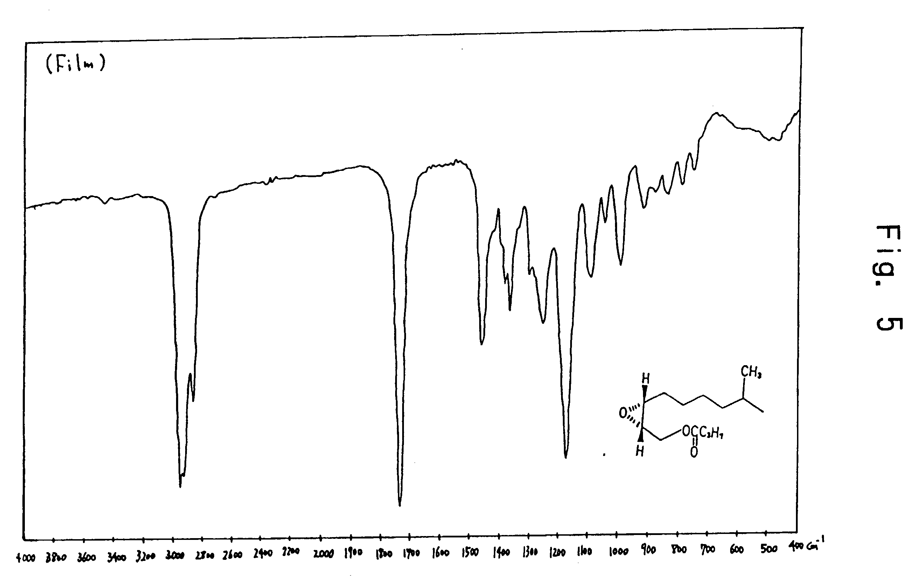

The infra red absorption spectra of (-)-(2S,3R)-1-butyroxy-2,3-epoxy-8-methylnonane showed the absorption at wavelengths (cm⁻¹ of 2,950(s), 2,930(s), 2,860(s), 1,740(s), 1,460(m), 1,360(m), 1,180(s), and 1,080(w) (see Fig. 5).

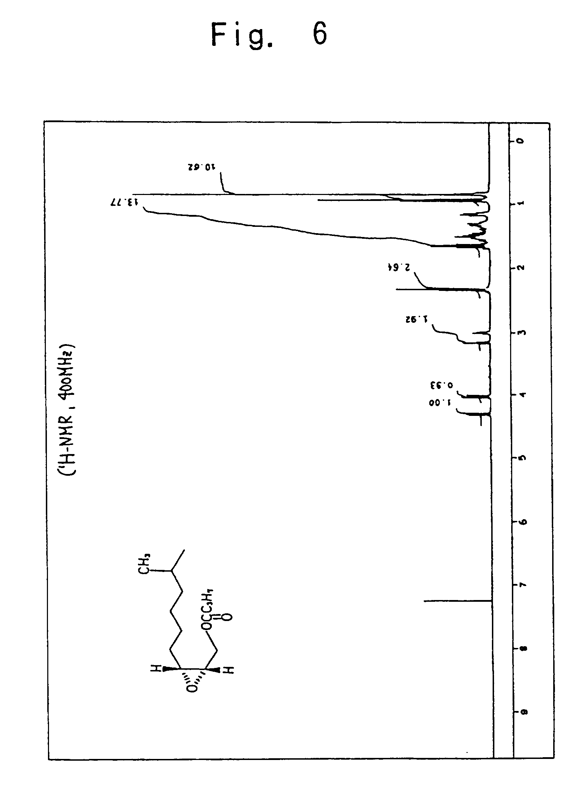

The δ values of the proton nuclear magnetic resonance spectra ¹H-NMR (CDC1₃, 400 MHz) were 0.85 (6H, d, J = 4.9Hz, 0.94 (3H, t, J = 2.7Hz), 1.14 through 1.69 (11H, m), 2.33 (3H, d, J = 7.3Hz), 2.99 (1H, m), 3.16 (1H, m), 4.03 (1H, m) and 4.31 (1H, m) (see Fig. 6).

3.6g of (-)-(2S,3R)-1-butyroxy-2,3-epoxy-8-methylnonane was hydrolized with potassium hydroxide/methanol to yield 2.4g of (-)-(2S,3R)-2,3-epoxy-8-methyl-1-nonanol.

### Example 4

Toluene (50ml), 2,3-epoxy-8-methyl-1-nonanol (5g), and n-valeric acid anhydride (3.2g) were mixed in a 100ml Mayer flask.

Lipase originated from pig pancreas (5g) [Trade name : Pancreatin F, Maker : Amano Pharmaceutical] was added to the mixed solution and stirred with a magnetic stirrer at 25°C for three hours for conclusion of the reaction.

After the solution was filtered, the filtrate was concentrated with a rotary evaporator and the solvent was removed.

The oily substance thus obtained was separated by silica gel column chromatography with hexane/ethyl acetate as eluate to yield 3.8g of (-)-(2S,3R)-1-valeroxy-2,3-epoxy-8-methylnonane (optical purity : 90% e.e) and 2.2g of (+)-(2R,3S)-2,3-epoxy-8-methyl-1-nonanol (optical purity: 92% e.e).

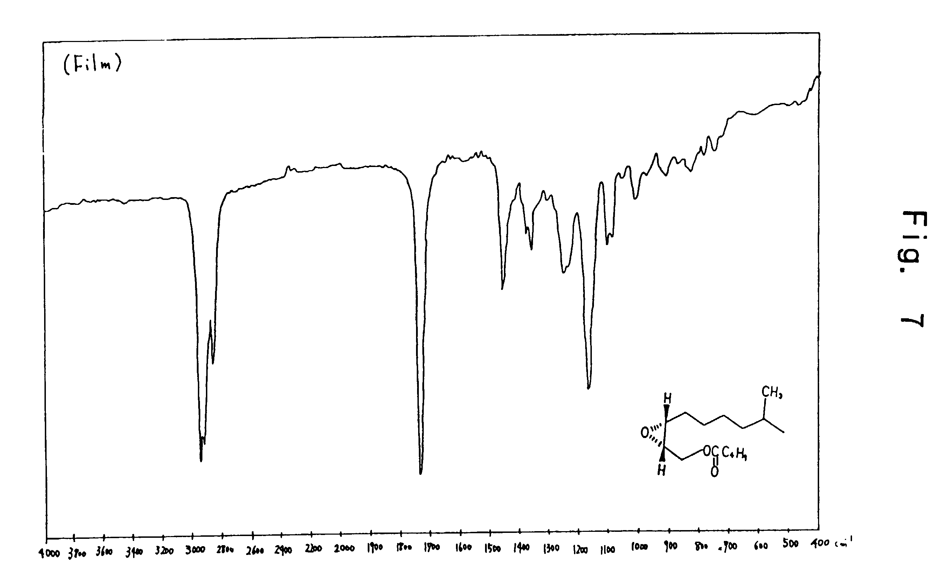

The infra red absorption spectra of (-)-(2S,3R)-1-valeroxy-2,3-epoxy-8-methylnonane showed the absorption at wavelengths (cm⁻¹ of 2,950(s), 2,930(s), 2,850(s), 1,740(s), 1,460(m), 1,360(w), 1,160(s), and 1,100(w) (see Fig. 7).

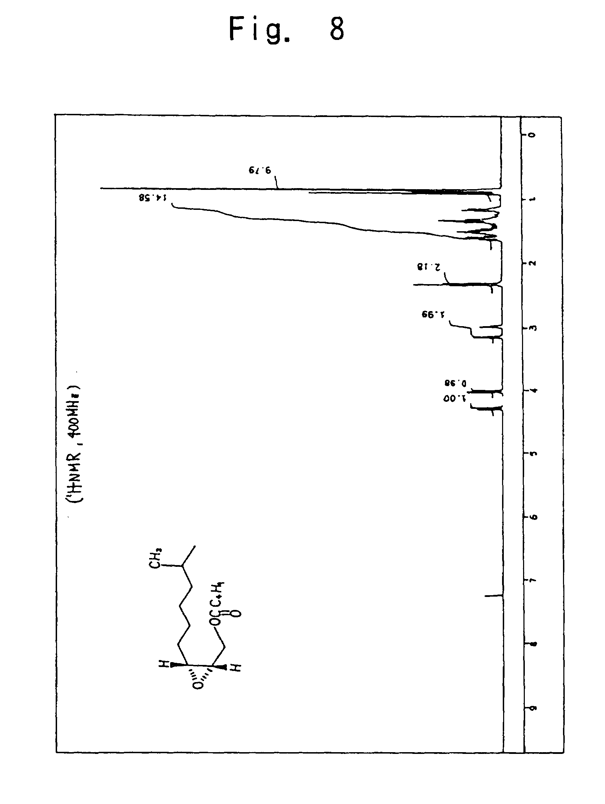

The δ values of the proton nuclear magnetic resonance spectra ¹H-NMR (CDC1₃, 400 MHz) were 0.85 (6H, d, J = 6.6Hz, 0.90 (3H, t, J = 7.3Hz), 1.13 through 1.64 (13H, m), 2.34 (2H, t, J = 7.3Hz), 2.98 (1H, m), 3.15 (1H, m), 4.02 (1H, m) and 4.29 (1H, m) (see Fig. 8).

3.8g of (-)-(2S,3R)-1-valeroxy-2,3-epoxy-8-methylnonane was hydrolized with potassium hydroxide/methanol to yield 2.3g of (-)-(2S,3R)-2,3-epoxy-8-methyl-1-nonanol.

### Example 5

Toluene (50ml), 2,3-epoxy-8-methyl-1-nonanol (5g) and caproic acid anhydride (3.2g) were mixed in a 100ml Mayer flask.

Lipase originated from pig pancreas (5g) [Trade name : Pancreatin F, Maker : Amano Pharmaceutical] was added to the mixed solution and stirred with a magnetic stirrer at 25°C for three hours for conclusion of the reaction.

After the solution was filtered, the filtrate was concentrated with a rotary evaporator and the solvent was removed.

The oily substance thus obtained was separated by silica gel column chromatography with hexane/ethyl acetate as eluate to yield 4.1g of (-)-(2S,3R)-1-capryloxy-2,3-epoxy-8-methylnonane (optical purity : 90% e.e) and 2.2g of (+)-(2R,3S)-2,3-epoxy-8-methyl-1-nonanol (optical purity: 92% e.e).

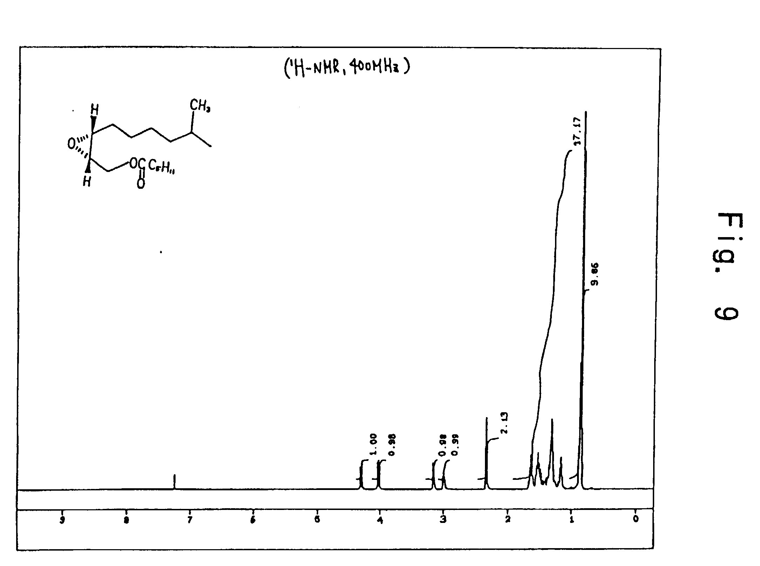

The infra red absorption spectra of (-)-(2S,3R)-1-capryloxy-2,3-epoxy-8-methylnonane showed the absorption at wavelengths (cm⁻¹ of 2,950(s), 2,920(s), 2,850(s), 1,740(s), 1,460(m), 1,360(w), 1,160(s), and 1,100(w) (see Fig. 9).

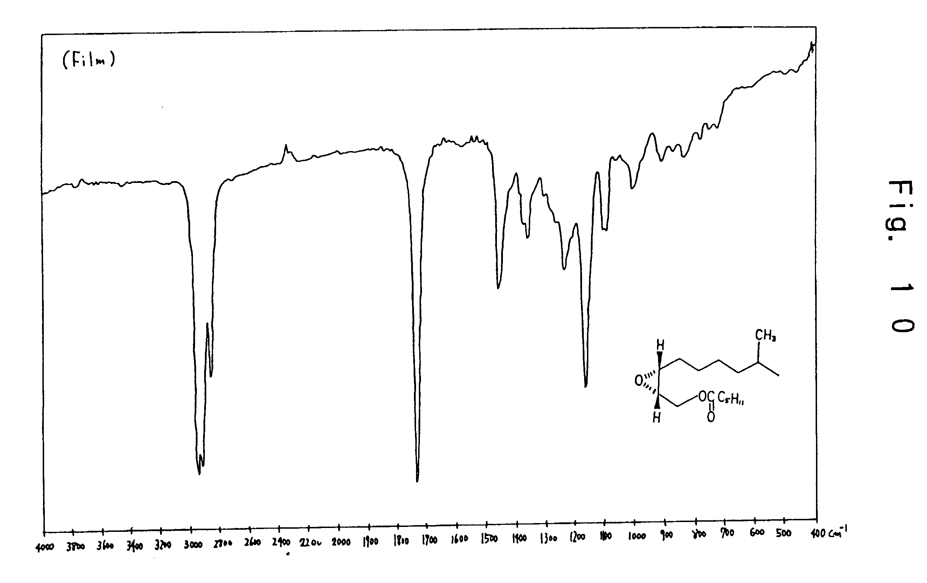

The δ values of the proton nuclear magnetic resonance spectra ¹H-NMR (CDC1₃, 400 MHz) were 0.85 (6H, d, J = 6.6Hz, 0.88 (3H, t, J = 6.8Hz), 1.13 through 1.64 (15H, m), 2.34 (2H, t, J = 7.3Hz), 2.99 (1H, m), 3.15 (1H, m), 4.02 (1H, m) and 4.30 (1H, m) (see Fig. 10).

4.1g of (-)-(2S,3R)-1-capryloxy-2,3-epoxy-8-methylnonane was hydrolized with potassium hydroxide/methanol to yield 2.3g of (-)-(2S,3R)-2,3-epoxy-8-methyl-1-nonanol.

## Claims

1. A process for preparing optically active epoxy alcohol, which is characterised in that it comprises the steps of:
adding carboxylic anhydride to racemic epoxy alcohol of formula 1 in the presence of a hydrolase in an organic solvent;
esterifying (-)-form of the epoxy alcohol preferentially to give an epoxy ester of formula 2; separating optically active epoxy ester of formula 2 from optically active epoxy alcohol of formula 3; and hydrolysing said optically active epoxy ester of formula 2 to yield optically active epoxy alcohol of formula 4;
whereby said optically active epoxy alcohol of formula 4 is synthesised in two steps, a first step being a hydrolyase enzyme reaction to prepare an optically active ester from a racemic alcohol and a second step being a hydrolysis reaction to prepare optically active alcohol from said optically active ester. 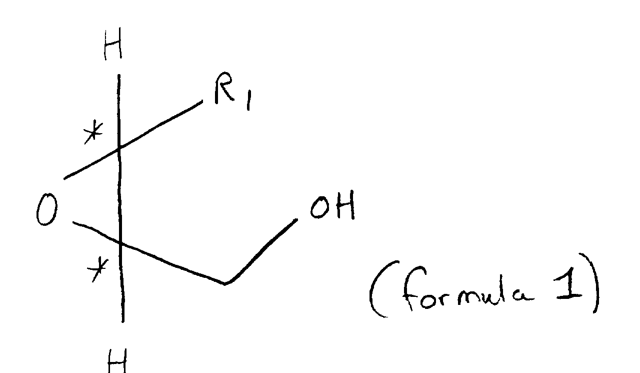 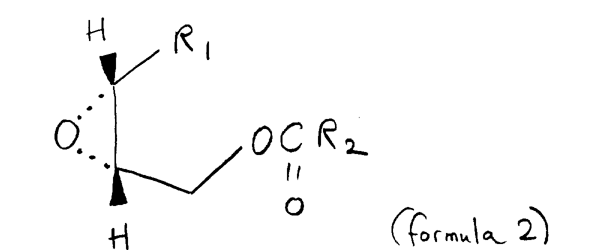 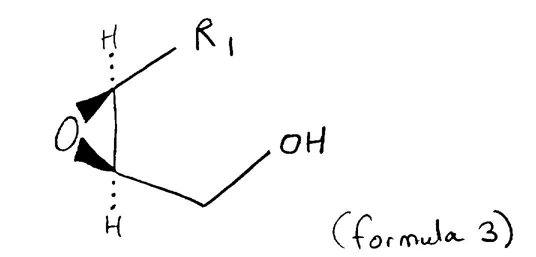 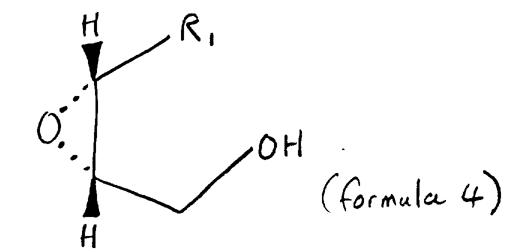 (Here R₁ and R₂ denote straight-chain or branched alkyl groups containing 1 through 20 carbon atoms. * denotes asymmetric carbon atoms).

2. A process for preparing optically active epoxy alcohol, in accordance with claim 1, wherein said epoxy alcohol (formula 1) is 2,3-epoxy-8-methyl-1-nonanol.

3. A process for preparing optically active epoxy alcohol, in accordance with claim 1, wherein said epoxy alcohol (formula 4) is 2,3-epoxy-8-methyl-1-nonanol.

4. A process for preparing optically active epoxy alcohol, in accordance with any one of claims 1 to 3, wherein said hydrolase is lipase.

## Patentansprüche

1. Verfahren zur Herstellung eines optisch aktiven Epoxyalkohols,
dadurch **gekennzeichnet**,
daß es folgende Stufen aufweist:
Zugabe eines Carbonsäureanhydrids zu einem racemischen Epoxyalkohol der Formel 1 in Anwesenheit von einer Hydrolase in einem organischen Lösungsmittel,
Veresterung der (-)-Form des Epoxyalkohols zu vorwiegend einem Epoxyester der Formel 2,
Abtrennen des optisch aktiven Epoxyesters der Formel 2 vom optisch aktiven Epoxyalkohol der Formel 3 und Hydrolyse des optisch aktiven Epoxyesters der Formel 2 zu einem optisch aktiven Epoxyalkohol der Formel 4,
wobei dieser optisch aktive Epoxyalkohol der Formel 4 in zwei Stufen synthetisiert wird, nämlich einer ersten Stufe, bei der es sich um eine Hydrolase-Enzym-Reaktion zur Herstellung eines optisch aktiven Esters aus einem racemischen Alkohol handelt, und einer zweiten Stufe, bei der es sich um eine Hydrolysereaktion zur Herstellung eines optisch aktiven Alkohols aus dem optisch aktiven Ester handelt. 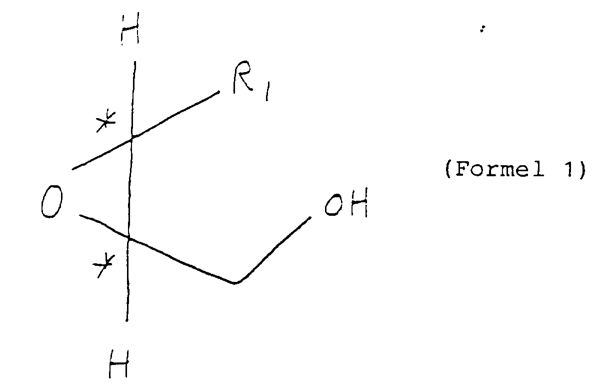 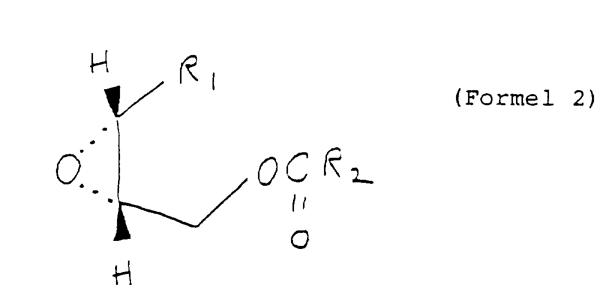 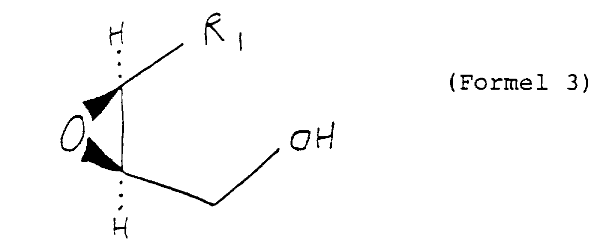 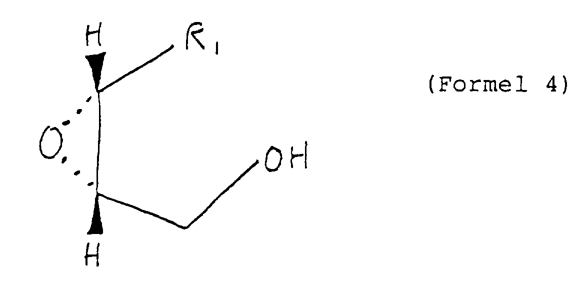 (dabei bedeuten R₁ und R₂ geradkettige oder verzweigte Alkylgruppen mit 1 bis 20 Kohlenstoffatomen; * bezeichnet asymmetrische Kohlenstoffatome).

2. Verfahren zur Herstellung eines optisch aktiven Epoxyalkohols nach Anspruch 1, bei dem der Epoxyalkohol der Formel 1 2,3-Epoxy-8-methyl-1-nonanol ist.

3. Verfahren zur Herstellung eines optisch aktiven Epoxyalkohols nach Anspruch 1, bei dem der Epoxyalkohol der Formel 4 2,3-Epoxy-8-methyl-1-nonanol ist.

4. Verfahren zur Herstellung eines optisch aktiven Epoxyalkohols nach einem der Ansprüche 1 bis 3, bei dem die Hydrolase Lipase ist.

## Revendications

1. Procédé pour préparer un époxy alcool optiquement actif, qui se caractérise en ce qu'il comporte les étapes consistant à :
ajouter un anhydride (d'acide) carboxylique à de l'époxy alcool racémique de formule 1, en présence d'une hydrolase dans un solvant organique;
estérifier la forme (-) de l'époxy alcool préférentiellement pour obtenir un époxy ester de formule 2;
séparer l'époxy ester optiquement actif de formule 2 de l'époxy alcool optiquement actif de formule 3; et
hydrolyser ledit époxy ester optiquement actif de formule 2 pour obtenir de l'époxy alcool optiquement actif de formule 4;
de sorte que ledit époxy alcool optiquement actif de formule 4 est synthétisé en deux étapes, une première étape étant une réaction d'une enzyme de type hydrolase pour préparer un ester optiquement actif à partir d'un alcool racémique, et une seconde étape étant une réaction d'hydrolyse pour préparer, à partir dudit ester optiquement actif, l'alcool optiquement actif. 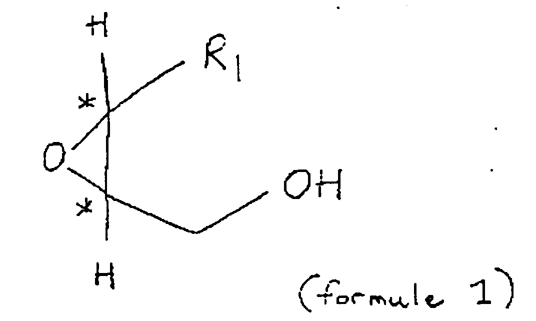 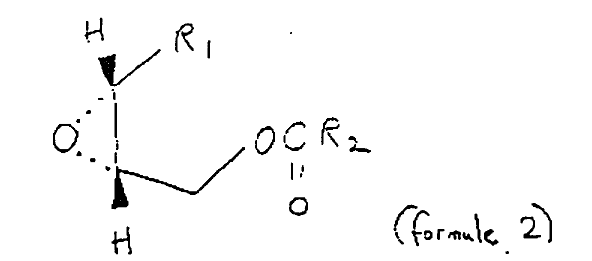 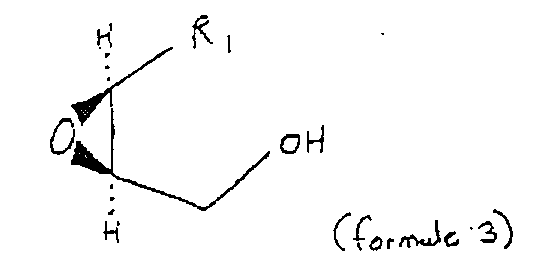 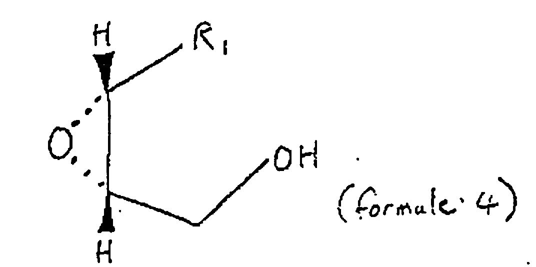 (les symboles R₁ et R₂ représentent ici les groupes alkyles linéaires ou ramifiés contenant 1 à 20 atomes de carbone; * désigne des atomes de carbone asymétrique).

2. Procédé pour préparer de l'époxy alcool optiquement actif, selon la revendication 1, dans lequel ledit époxy alcool (formule 1) est du 2,3-époxy-8-méthyl-1-nonanol.

3. Procédé pour préparer de l'époxy alcool optiquement actif, selon la revendication 1, dans lequel ledit époxy alcool (formule 4) est du 2,3-époxy-8-méthyl-1-nonanol.

4. Procédé pour préparer l'époxy alcool optiquement actif, selon l'une quelconque des revendications 1 à 3, dans lequel ladite hydrolase est la lipase.
